# EUROPÄISCHE PATENTSCHRIFT

(11) **EP 2 120 601 B1**
(45) Veröffentlichungstag und Bekanntmachung des Hinweises auf die Patenterteilung: **27.10.2010**
(21) Anmeldenummer: 08707573.5
(22) Anmeldetag: 06.02.2008
(51) Int. Cl.: A23L 1/05, A23L 1/09, A61K 9/50

(54) **FLIESSFÄHIGE GELATINEZUSAMMENSETZUNG**
FREE-FLOWING GELATIN COMPOSITION
COMPOSITION DE GÉLATINE À ÉCOULEMENT LIBRE

(30) Priorität: 07.02.2007 DE 102007007307
(43) Veröffentlichungstag der Anmeldung: 25.11.2009
(73) Patentinhaber: GELITA AG, 69412 Eberbach (DE)
(72) Erfinder: DICK, Eberhard, 69151 Neckargemünd (DE); WALTER, Simone, 69483 Wald-Michelbach (DE)
(74) Vertreter: Wössner, Gottfried
(86) Internationale Anmeldenummer: PCT/EP2008/000907
(87) Internationale Veröffentlichungsnummer: WO 2008/095688

(56) Entgegenhaltungen:
- WO-A-03/026439
- WO-A-2005/079819
- US-A1- 2004 013 732

## Beschreibung

Die Erfindung betrifft eine neuartige fließfähige Gelatinezusammensetzung, insbesondere zur Verwendung als Lebensmittelvorprodukt. Die neuartige fließfähige Gelatinezusammensetzung behält ihre Fließfähigkeit insbesondere auch bei Temperaturen unter 30°C, beispielsweise bei 25 °C.

Die industrielle Verarbeitung von Gelatine, insbesondere in der Lebensmittelindustrie, nutzt insbesondere die Gelbildungseigenschaften gelöster Gelatine bei der Herstellung von Lebensmittelprodukten. Traditionell wird hierzu Gelatine in trockenem Zustand, insbesondere in Pulverform, als Ausgangsprodukt eingesetzt, welches unter Zusatz von Wasser und unter Erwärmen in Lösung gebracht wird. Ein gemeinsames Lösen der Gelatine mit eventuell weiteren Zutaten ist in der Regel kaum möglich, da aufgrund der Konkurrenz um das zur Verfügung stehende Wasser der Gelatineanteil kaum noch gelöst werden kann. Deshalb wird zunächst eine Lösung des Gelatineanteils hergestellt und die Lösung dann mit den restlichen Bestandteilen des Lebensmittelproduktes vermischt, um dieses dann unter Kühlen gelieren zu lassen.

Im Hinblick auf die nicht besonders gut ausgeprägte Benetzbarkeit und die fehlende Kaltwasserlöslichkeit von Gelatine, insbesondere in Pulverform, stellt sich das Mischen mit Wasser und die Überführung in flüssige Form als ein aufwändiger Teil des Herstellungsprozesses dar. Dies hängt insbesondere auch damit zusammen, dass die Gelatinepartikel beim Einrühren in Flüssigkeiten leicht zusammenkleben und verklumpen, sodass die gleichmäßige Quellung der Gelatinepartikel zu Gelatinegelpartikeln und deren Auflösung in der Flüssigkeit verzögert wird.

Übermäßiges Rühren um die Klumpenbildung zu vermeiden kann hingegen zu starker Schaumbildung führen, was den Produktionsprozess ebenfalls stark stört.

Diese so genannte Instantgelatine ist zwar kalt löslich und kann direkt mit allen Zutaten vermischt verarbeitet werden, ohne zuvor diese spezielle Gelatine separat auflösen zu müssen. Jedoch lassen sich mit solchen Gelatineprodukten keine echten Gele, sondern nur gelartige Strukturen erzeugen, welche bei gleicher Dosierung sehr viel geringere Gelstärken aufweisen als ein Gel, welches aus einer vergleichbaren Pulvergelatine konventionell hergestellt ist.

Eine weitere Beschränkung der Verwendbarkeit der Instantgelatine ist die um ein Vielfaches höhere Gefahr der Klumpenbildung im Vergleich zu Pulvergelatine, weshalb die Verwendung von Instantgelatine bei vielen Anwendungen alternativ zu Pulvergelatine nicht möglich ist.

Eine gewisse Abhilfe wird durch das Untermischen von zuckerhaltigen Trägerstoffen oder von Gelatinehydrolysat unter die Instantgelatine gefunden, wobei die zuckerhaltigen Trägerstoffe bzw. das Gelatinehydrolysat verwendet wird, um die Gelatinepartikel des Gelatinepulvers zu agglomerieren. Beim Einrühren der agglomerierten Partikel in Flüssigkeiten lösen sich die Trägerstoffe bzw. das Gelatinehydrolysat schneller auf als die Gelatinepartikel selbst und lassen diese dann fein verteilt in der Flüssigkeit zurück. Außerdem erleichtern die Trägerstoffe bzw. das Gelatinehydrolysat die anfängliche Benetzung. Das Problem der verminderten Gelfestigkeit lässt sich damit allerdings nicht lösen, es sei denn die Mischung wird als Ganzes über den Schmelzpunkt der Gelatine erhitzt, um somit eine echte Lösung herzustellen.
Aufgrund des aufwändigen Herstellungsprozesses sind solche Instantgelatineprodukte teurer als die Pulvergelatine, was sich insbesondere bei kostensensitiven Lebensmittelprodukten ungünstig auf die Kostensituation auswirkt.

Aufgabe der vorliegenden Erfindung ist es, ein kostengünstiges Gelatineprodukt vorzuschlagen, welches vereinfacht industriell weiterverarbeitbar ist.

Diese Aufgabe wird durch eine neuartige fließfähige Gelatinezusammensetzung gelöst, welche eine wässrige Flüssigkeit mit darin dispergierten Gelatinegelpartikeln und/oder darin gelöstem Gelatinehydrolysat und einen Zuckerbestandteil umfasst, wobei die Summe der Gehalte an Gelatine, Gelatinehydrolysat und Zuckerbestandteil so gewählt ist, dass die Wasseraktivität (aw-Wert) der Zusammensetzung kleiner oder gleich 0,97 ist.

Die Verarbeitung von Gelatine im industriellen Maßstab, die in einem Trocknungsprozess aus einer wässrigen Lösung gewonnen wird, verlangt in der Regel als ersten Verarbeitungsschritt das erneute Auflösen dieser Trockenprodukte. Von Vorteil wäre es daher, dieses erneute Auflösen zu vermeiden und Gelatine oder Gelatinehydrolysate direkt ohne Trocknung an die Verwender auszuliefern. Reine Gelatine und Gelatinehydrolysate sind aber, abgesehen von nur sehr kurzen Zwischenlagerungen, nur in trockenem Zustand lagerstabil. Daher müssen diese Produkte zumindest nach heutigem Stand der Technik direkt nach ihrer Herstellung, wo sie noch in flüssiger Form vorliegen, umgehend getrocknet werden um sie lager- und transportfähig zu machen.

Die Gründe, die eine Flüssiglagerung bzw. einen Transport bei Temperaturen unter 30°C unmöglich machen, liegen zum einen in der geringen mikrobiologischen Stabilität von solchen Lösungen und zum anderen, im Falle von Gelatine in der Tatsache, dass Gelatine bei diesen Temperaturen als festes Gel vorliegt. Höhere Lagertemperaturen, die sowohl das Verderbsrisiko minimieren als auch das Gelieren von Gelatine verhindern sind keine Lösung, da unter diesen Bedingungen massive thermische Schädigungen auftreten, welche das Produkt für eine weitere Verwendung ungeeignet machen, so dass auch diese Alternative allenfalls für kurzfristige Zwischenlagerungen genutzt werden kann.

Eine weitere Alternative, zumindest bei Gelatinehydrolysaten, die auch bei Temperaturen unter 30°C noch flüssig sind, wäre natürlich die Verwendung von Konservierungsmitteln. Die Verwendung solcher Stoffe ist lebensmittelrechtlich gesehen weltweit nicht unproblematisch und stößt bei vielen Anwendern auf mangelnde Akzeptanz.

Die erfindungsgemäßen fließfähigen Gelatinezusammensetzungen lassen sich problemlos in Tankwagen transportieren, oft ohne dass dieser beheizt werden muss, und weisen überraschenderweise auch die notwendige mikrobiologische Stabilität für den Transport und die Lagerung auf, welche insbesondere dadurch erreicht wird, dass die Summe der Gehalte an Gelatine, Gelatinehydrolysat und Zuckerbestandteil so gewählt ist, dass die Wasseraktivität der Zusammensetzung kleiner oder gleich 0,97 ist.

Eine Wasseraktivität kleiner oder gleich 0,97 bedeutet, dass der Wasserdampfpartialdruck an der Oberfläche der fließfähigen Gelatinezusammensetzung kleiner oder gleich dem 0,97-fachen des Wasserdampfpartialdruckes ist, der sich direkt über der Oberfläche von reinem Wasser einstellt.

Die dadurch erreichte mikrobiologische Stabilität der fließfähigen Gelatinezusammensetzung ist ausreichend für die Herstellung, Lagerung, den Transport und Bevorratung auf Seiten des industriellen Verwenders.

Aufgrund der Fähigkeit der Gelatine in großem Umfang Wasser aufzunehmen, lässt sich über die Dosierung der Gelatinegelpartikel in der wässrigen Flüssigkeit alleine eine Wasseraktivität kleiner oder gleich 0,97 in der Praxis nicht erreichen. Hierbei ist der Zusatz von einem oder mehreren Zuckerbestandteilen eine optimale Problemlösung, da Zuckerbestandteile selbst häufig in Lebensmittelrezepturen enthalten sind und seitens der industriellen Verwender der bereits in der fließfähigen Gelatinezusammensetzung angelieferte Gehalt bei der Rezeptierung im Rahmen der Weiterverarbeitung problemlos berücksichtigt werden können.

Die erfindungsgemäße neuartige fließfähige Gelatinezusammensetzung lässt sich einfach im industriellen Prozess weiterverarbeiten, indem die fließfähige Zusammensetzung lediglich erwärmt werden muss, um die darin dispergierten Gelatinepartikel vollends zu schmelzen und die Gelatine so in Lösung zu bringen. Beim Abkühlen erhält man dann, wie von der Verarbeitung der herkömmlichen Pulvergelatine gewohnt, feste Gelstrukturen.

Im Falle der ausschließlichen Verwendung von Gelatinehydrolysat ist ein Erwärmen nicht notwendig, da dieses schon mit den anderen Bestandteilen der erfindungsgemäßen Zusammensetzung eine molekulardisperse, homogene Mischung bildet.

Der Gehalt an Gelatinehydrolysat ist nicht auf einen löslichen Anteil beschränkt. Vielmehr sind im Rahmen der Erfindung auch solche Zusammensetzungen zu nennen bei denen gelöstes und ungelöstes Gelatinehydrolysat neben einander vorliegt. Die ungelösten Anteile an Gelatinehydrolysat liegen dann bevorzugt in der erfindungsgemäßen Zusammensetzung dispergiert vor.

Erfindungsgemäß eignen sich als Zuckerbestandteile Saccharide, insbesondere Mono-, Di- und Oligosaccharide, insbesondere Saccharose, Glucose, Fructose, Glucosesirupe, Oligofructosesirupe, Dextrine und dergleichen.

Weitere geeignete Zuckerbestandteile sind Zuckeraustauschstoffe, insbesondere Alditole, wie z.B. Glycerin, Threit, Mannit, Isomalt, Lactit, Sorbit, Xylit, Erythrit, Arabit und Maltit, sowie Polydextrose.

Die vorgenannten Zuckerbestandteile lassen sich einzeln oder in beliebiger Abmischung in der erfindungsgemäßen Zusammensetzung verwenden.

Ein weiterer Vorteil bei der Verwendung der erfindungsgemäßen Zusammensetzung ist das vereinfachte Handling, da der relativ große Aufwand bei der Herstellung insbesondere von hoch konzentrierten Gelatinelösungen entfällt.

Gelatine in Form der Gelatinegelpartikel und Gelatinehydrolysat können in der fließfähigen Gelatinezusammensetzung nebeneinander in beliebiger Abmischung eingesetzt werden, wobei die Gelatine und auch das Gelatinehydrolysat alleine in der Zusammensetzung vorhanden sein kann.

Die erfindungsgemäße fließfähige Gelatinezusammensetzung lässt sich insbesondere kostengünstig herstellen, da die bei der Herstellung von Gelatinepulver oder Gelatinehydrolysatpulver notwendige Trocknung bis auf einen Wassergehalt von 10 Gew.-% unterbleiben kann. Insbesondere kann man im Falle des Gelatinebestandteils von einem Zwischenprodukt der Gelatineherstellung, den so genannte Gelatinenudeln, ausgehen, die ca. 30 Gew.-% Gelatinetrockensubstanz und ca. 70 Gew.-% Wasser enthalten. Diese Gelatinenudeln lassen sich mit einem Schneidwerk (Kutter) unter Kühlbedingungen (Temperatur unter 20 °C) in einfacher Weise zerkleinern, um ausreichend feine Gelatinegelpartikel herzustellen.

Diese Gelatinegelpartikel lassen sich mit dem Zuckerbeständteil und gegebenenfalls dem gewünschten Gelatinehydrolysatanteil vermischen, wodurch sich eine fließfähige Dispersion der Gelatinegelpartikel in einer flüssigen Matrix ergibt, die pumpfähig ist und so im industriellen Prozess einfach dosierbar verwendet werden kann.

Gelatinegelpartikel lassen sich dabei auch direkt mit dem Zuckerbestandteil in fester Form mischen, wobei sich schon während des Mischvorgangs eine pumpfähige Dispersion bildet, da aufgrund osmotischer Effekte ein Teil des in den Gelatinegelpartikeln gebundenen Wassers austritt und zum Lösen des Zuckerbestandteils zur Verfügung steht.

Bei der erfindungsgemäßen vorgegebenen Wasseraktivität von kleiner oder gleich 0,97 bleibt bei Temperaturen von ca. 20 °C eine mikrobiologische Stabilität für mindestens 2 bis 3 Wochen gewährleistet, die Einhaltung der üblichen hygienischen Bedingungen der Gelatineherstellung vorausgesetzt.

Auf der Kunden- oder Verarbeiterseite werden zu der fließfähigen Gelatinezusammensetzung nur noch die weiteren Rezepturbestandteile zudosiert und mit der fließfähigen Zusammensetzung vermischt, wobei diese Mischung dann unmittelbar über ein ohnehin notwendiges Kochsystem gefahren werden kann, um eine gelierende Gießlösung für beispielsweise das Lebensmittelprodukt, insbesondere Gummibonbons oder Gummibärchen zu erhalten. Herstellungsseitig wird bei der erfindungsgemäßen fließfähigen Gelatinezusammensetzung damit nicht nur ein Trocknungsschritt eingespart, sondern auch die Zwischenlagerung des Produktes vor dem Trocknungsschritt für weitere Laboruntersuchungen und das Mahlen und Mischen sowie das Abpacken des Gelatinepulvers. Die dem gegenüberstehenden zusätzlichen Kosten für das Zerkleinern der Gelatinenudeln, das Mischen z.B. mit Zucker und Glukosesirup sowie die Mehrkosten für den Transport sind damit leicht zu rechtfertigen.

Auf Seiten der Verarbeiter entfällt der komplette Schritt der Gelatinequellung und Auflösung, und die Dosierung und Mischung der erfindungsgemäßen fließfähigen Gelatinezusammensetzung mit den restlichen Rezepturbestandteilen gestaltet sich einfacher. Dies erbringt beim Verarbeiter nicht nur Einsparungen auf der apparativen Seite, sondern auch bei den Personalkosten, da die Verarbeitung der erfindungsgemäßen fließfähigen Gelatinezusammensetzung im Gegensatz zu der pulverförmigen Gelatine völlig unproblematisch vollautomatisch stattfinden kann.

Darüber hinaus kann bei den Verarbeitern, die Pulvergelatine häufig in Batchverfahren, die komplizierte Vorrats- und Verwiegesysteme benötigen, verarbeiten, ein kontinuierlich arbeitendes System eingesetzt werden, was bei bestehenden Anlagen durch entsprechende Umrüstung leicht aufgebaut werden kann.

Eine weitergehende mikrobielle Stabilisierung lässt sich durch ein Absenken des pH-Wertes erzielen, beispielsweise auf Werte kleiner 5, insbesondere ca. 3 bis 4,5, wobei hierfür bevorzugt Genusssäuren verwendet werden können. Der aw-Wert bleibt hiervon weitgehend unbeeinflusst.

Wird eine noch längere mikrobiologische Haltbarkeit der erfindungsgemäßen Gelatinezusammensetzung benötigt, empfiehlt es sich, die Wasseraktivität der Zusammensetzung auf 0,93 oder weniger herabzusetzen. Die Rezeptierung, d.h. die Anteile an Gelatinegelpartikel und/oder gelöstem Gelatinehydrolysat einerseits und Zuckerbestandteil andererseits, muss entsprechend angepasst werden.

Werden Gelatinegelpartikel als alleiniger Gelatinebestandteil in der fließfähigen Gelatinezusammensetzung verwendet, lässt sich der Gehalt an Gelatinegelpartikeln (ausgedrückt in Trockenmasse mit einem Wassergehalt von ca. 10 Gew.-%) im Bereich von 20 bis 40 Gew.-%, bezogen auf das Gesamtgewicht der Zusammensetzung, variieren.

Höhere Konzentrationen an Gelatine oder anders ausgedrückt Wassergehalte von 50 Gew.-% oder weniger (bei Gelatinehydrolysat basierenden Zusammensetzungen 35 Gew.-% oder weniger) sind einerseits technisch nur aufwändig zu realisieren und ergeben darüber hinaus Viskositäten, die die Pumpfähigkeit stark einschränken.

Wird überwiegend oder ausschließlich Gelatinehydrolysat als Gelatinebestandteil in der flüssigen Gelatinezusammensetzung verwendet, kann dessen Gehalt problemlos im Bereich von 20 bis 60 Gew.-% variiert werden.

Das mittlere Molekulargewicht des Gelatinehydrolysats wird vorzugsweise im Bereich von ca. 1.000 bis ca. 20.000 Da vorgegeben.

Die Gelatinegelpartikel weisen in gequollenem Zustand (d.h. mit maximalem Wassergehalt) bevorzugt eine mittlere Partikelgröße von ca. 0,01 bis 3 mm, insbesondere 0,1 bis 1 mm auf.

Da bei der Verwendung von Gelatinehydrolysat als Gelatinebestandteil in der erfindungsgemäßen Zusammensetzung höhere Gesamtprotein-Gehalte möglich sind, braucht der Gehalt des Zuckerbestandteils in solchen Zusammensetzungen nicht so hoch angesetzt werden, wie dies für Zusammensetzungen mit Gelatinegelpartikeln als Gelatinehauptbestandteil der Fall ist. Hier können Gehalte von ca. 10 Gew.-% oder mehr an Zuckerbestandteil bereits eine ausreichende mikrobiologische Stabilisierung herbeiführen.

Gelatinehydrolysate können für die erfindungsgemäßen Zusammensetzungen in Form von Lösungen verwendet werden, wie sie derzeit bereits bei der Herstellung von Hydrolysatpulver im Sprühtrocknungsprozess eingesetzt werden.

Diesen Lösungen werden der Zuckerbestandteil, gegebenenfalls Genusssäuren und je nach Kundenwunsch eventuell weitere Rezepturbestandteile zugegeben. Die notwendigen Misch- und Auflöseschritte lassen sich in einem breiten Temperaturbereich durchführen.

Ein typischer Vertreter für die erfindungsgemäß verwendeten Zuckerbestandteile der Gelatinezusammensetzung sind - wie schon erwähnt - die Saccharide, die in einer Rezeptur, die hauptsächlich auf Gelatinegelpartikel als Gelatinebestandteil abstellt, vorzugsweise in einer Menge von 30 Gew.-% oder mehr, bezogen auf die Gesamtzusammensetzung, eingesetzt werden.

Die Saccharide werden bevorzugt ausgewählt aus Mono-, Di- und/oder Oligosacchariden, wobei insbesondere Mono- und Disaccharide zum Einsatz kommen, in Zusammensetzungen, die als Lebensmittelvorprodukte bestimmt sind.

Soll die erfindungsgemäße Gelatinezusammensetzung in Rezepturen verwendet werden, die von Haus aus keine Saccharide enthalten, bieten sich als Alternative die erwähnten Zuckeraustauschstoffe, insbesondere Alditole, wie z.B. Glycerin oder andere Zuckeralkohole, Oligofructosesirupe, Polydextrose und Dextrine, insbesondere Weizendextrin, an.

Solche Zusammensetzungen sind insbesondere für die Herstellung von diätetischen, zuckerreduzierten Produkten geeignet, insbesondere für die Herstellung von zuckerreduzierten Gummibonbons.

Im Hinblick auf die Pumpfähigkeit der erfindungsgemäßen fließfähigen Gelatinezusammensetzung wird bevorzugt, wenn die Zusammensetzung eine Viskosität von höchstens ca. 20.000 cP, weiter bevorzugt höchstens 10.000 cP aufweist. Allerdings sind auch Zusammensetzungen mit Viskositäten von ca. 100.000 cP fließfähig und mit herkömmlicher Ausrüstung lebensmitteltechnologisch verarbeit-, pump- und dosierbar.

Diese und weitere Vorteile der Erfindung werden im Folgenden anhand der Beispiele noch näher erläutert.

Wo in den folgenden Beispielen gequollene Gelatinegelpartikel verwendet werden, stammen diese aus einem Zwischenschritt der Gelatineproduktion, in dem sogenannte Gelatinenudeln anfallen mit einem Wassergehalt von ca. 70 Gew.-%. Diese Nudeln wurden in einem sogenannten Kutter wie weiter oben beschrieben auf die in den einzelnen Beispielen angegebenen Partikelgrößen zerkleinert.

### Beispiel 1: Gelatinedispersion

### Beispiel A

| | |
|---|---|
| 61,4 Gew.-% | gequollene Gelatinegelpartikel (Trockensubstanz ca. 30 Gew.-%) mittlere Partikelgröße 0,4 mm; Bloom = 260; Gelatine Typ A |
| 28,0 Gew.-% | Saccharose |
| 10,60 Gew.-% | Glukosesirup (78 Gew.-%ig) |

Die Rezepturbestandteile lassen sich ohne Wasserzusatz miteinander mischen und ergeben eine fließfähige erfindungsgemäße Zusammensetzung mit einem Wassergehalt von ca. 43,4 Gew.-%. Der aw-Wert beträgt 0,97.

Diese erfindungsgemäße Gelatinezusammensetzung weist bei 20 °C eine Viskosität von ca. 2500 cP auf.

### Beispiel B

| | |
|---|---|
| 60,00 Gew.-% | gequollene Gelatinegelpartikel (Trockensubstanz ca. 30 Gew.-%), mittlere Partikelgröße 0,4 mm; Bloom = 280; Gelatine Typ A |
| 40,00 Gew.-% | Saccharose |

Die Rezepturbestandteile lassen sich ohne Wasserzusatz miteinander mischen und ergeben eine fließfähige erfindungsgemäße Zusammensetzung mit einem Wassergehalt von ca. 42 Ges.-%. Der aw-Wert beträgt 0,963. Diese erfindungsgemäße Gelatinezusammensetzung weist bei 20 °C eine Viskosität von ca. 14000 cP auf.

Sie eignet sich insbesondere als Lebensmittelvorprodukt bei der Herstellung von Gummibärchen oder Gummibonbons.

Der Verarbeiter braucht zu der erfindungsgemäßen Gelatinezusammensetzung lediglich noch Glukosesirup, Saccharose und Aromastoffe sowie gegebenenfalls Farbstoffe zuzufügen und diese Mischung über ein Kochsystem fahren, um in einfacher Weise eine fertige Gießlösung zu bekommen, die in übliche Gießformen abgegossen werden kann.

### Beispiel 2: Gelatinedispersion zuckerfrei

| | |
|---|---|
| 50 Gew.-% | gequollene Gelatinegelpartikel (Trockensubstanz ca. 30 Gew.-%), mittlere Partikelgröße 0,3 mm, Bloom = 240; Gelatine Typ A |
| 25 Gew.-% | Weizendextrin (erhältlich als Nutriose^{®} von Roquette Frères, Frankreich) |
| 25 Gew.-% | Polydextrose |

Der Wasseranteil dieser Rezeptur liegt bei 36,5 Gew.%, der aw-Wert beträgt 0,95.

Diese erfindungsgemäße Gelatinezusammensetzung weist bei 20°C eine Viskosität von ca. 6000 cP auf.

Sie eignet sich insbesondere als Vorprodukt zur Herstellung von zuckerreduzierten bzw. zuckerfreier Süßwaren.

### Beispiel 3: Flüssighydrolysat

| | |
|---|---|
| 40 Gew.-% | Gelatinehydrolysat (Trockensubstanz), mittleres Molekulargewicht = 3000 Da |
| 16 Gew.-% | Saccharose |

| | |
|---|---|
| [1 Gew.-% Zitronen-Säure zur Absenkung des pH auf ca. pH 4,5; optional] Rest Wasser | |

Der aw-Wert beträgt 0,942.

Diese erfindungsgemäße Gelatinezusammensetzung weist bei 20°C eine Viskosität von ca. 1120 cP auf.

Diese erfindungsgemäße Gelatinezusammensetzung lässt sich ebenfalls als Lebensmittelvorprodukt einsetzen, z.B. bei der Herstellung von proteinangereicherten Gummibonbons oder der Riegelherstellung.

Es versteht sich, dass die Rezepturen der Beispiele 1 bis 3 so abgewandelt werden können, dass der Gelatinegehalt z.T. durch Gelatinehydrolysat (Beispiele 1 und 2) bzw. durch Gelatinegelpartikel (Beispiel 3) gebildet wird.

Wird in Beispiel 1 zusätzlich Gelatinehydrolysat verwendet, hat man bereits ein Vorprodukt für die Herstellung proteinangereicherter Lebensmittel, z.B. Gummibonbons oder Marshmallows.

Die folgenden Tabellen 1 und 2 zeigen wie man mit unterschiedlichen Gehalten der Bestandteile der erfindungsgemäßen Zusammensetzung einfach den geforderten aw-Wert einstellen kann.

**Tabelle 1**

| Gew.-% Gelatine (Bloom = 220; Typ A) | Gew.-% Zucker (Saccharose) | Gew.-% TS Mix | aw-Wert |
|---|---|---|---|
| 25,0 % | 0,0 % | 25,0 % | 0,994 |
| 19,2 % | 23,1 % | 42,3 % | 0,970 |
| 24,4 % | 25,9 % | 50,3 % | 0,966 |
| 29,3 % | 28,6 % | 57,9 % | 0,959 |
| 34,7 % | 33,3 % | 68,0 % | 0,932 |

**Tabelle 2**

| Gew.-% Hydrolysat TS (MW = 3000 Da) Ausgangsprodukt | Gew.-% Zucker 100 (Saccharose) | Gew.-% TS Mix | aw-Wert |
|---|---|---|---|
| 51,5 % | 0,0 % | 51,5 % | 0,972 |
| 42,9 % | 18,0 % | 60,9 % | 0,935 |
| 34,3 % | 34,1 % | 68,4 % | 0,883 |
| 28,6 % | 43,3 % | 71,9 % | 0,848 |
| 25,7 % | 49,7 % | 75,4 % | 0,808 |

Aus den Tabellen ist ebenfalls ersichtlich, dass die erfindungsgemäße Gelatinezusammensetzung (Mix) mit Vorteil sehr hohe Anteile an Trockensubstanz (TS) aufweist, wobei nicht nur der Vorteil der mikrobiologischen Stabilität erzielt wird, sondern die Weiterverarbeitung zum Fertigprodukt energetisch günstig erfolgen kann, da nur verhältnismäßig geringe Wasseranteile in der Produkttrocknung ausgetrieben werden müssen.

### Beispiel 4:

Um die Löslichkeit der Gelatinegelpartikel bei der Weiterverarbeitung noch weiter zu verbessern, kann man schon bei der Herstellung der Gelatinegelpartikel, der in der Vorstufe vorkommenden Gelatinelösung zusätzlich noch einen Zuckerbestandteil, z.B. Zucker, zugeben, der dann in der Lösung gelöst und molekulardispers verteilt ist. Die daraus hergestellten Gelteilchen werden wie die nur aus Gelatine und Wasser bestehenden Gelteilchen mit Zucker, Glukosesirup etc. vermischt, um daraus eine stabile Dispersion herzustellen.

### Rezepturbeispiel:

In einer in der Gelatineproduktion als Zwischenprodukt anfallenden ca. 30 Gew.-%igen Gelatinelösung wird Zucker aufgelöst so dass die Lösung folgende Zusammensetzung hat:

| | |
|---|---|
| Wasser | 54 Gew.-% |
| Gelatine (TS) | 23 Gew.-% |
| Zucker | 23 Gew.-% |

anschließend wird die Lösung abgekühlt und geliert (analog zur normalen Verarbeitung von Gelatine) und die hieraus hergestellten Gelpartikel mit Zucker und Glucosesirup vermischt um eine erfindungsgemäße Dispersion herzustellen, die beispielsweise folgende Zusammensetzung hat:

| | | |
|---|---|---|
| Gelatine (TS) | 11,7 Gew.-% | |
| Zucker | 26,6 Gew.-% | |
| Glucosesirup | 24,0 Gew.-% | (78 % TS) |
| Rest Wasser | | |

Der Zuckergehalt von 26,6 Gew.-% setzt sich zusammen aus 13,3 Gew.-% in den Gelatinegelpartikeln enthaltenem Zucker und 13,3 Gew.-% als Trockensubstanz in Reinform zugegebenem Zucker.

Die Viskosität dieser erfindungsgemäßen Zusammensetzung beträgt 3.000 cP. Der erzielte aw-Wert beträgt 0,961.

### Beispiel 5: Gelatinehydrolysatzusammensetzung A

| | |
|---|---|
| 37,5 Gew.-% | Gelatinehydrolysat (Trockensubstanz) mittleres Molekulargewicht = 3000 Da |
| 25,0 Gew.-% | Nutriose (95 Gew.-% Trockensubstanz) |
| Rest | Wasser |

In einer solchen Zusammensetzung liegt ein Teil des Gelatinehydrolysats in gelöster Form und ein weiterer Teil in fester, dispergierter Form vor.

Als aw-Wert ergibt sich 0,935. Die Viskosität bei 20 °C beträgt ca. 24.570 cP.

### Beispiel 6: Gelatinehydrolysatzusammensetzung B

Zu der Zusammensetzung von Beispiel 5 wurden weitere Anteile des Gelatinehydrolysats in Pulverform zugegeben, so dass die folgende Zusammensetzung resultierte:

| | |
|---|---|
| 43,3 Gew.-% | Gelatinehydrolysat (Trockensubstanz) mittleres Molekulargewicht = 3000 Da |
| 22,2 Gew.-% | Nutriose (95 Gew.-% Trockensubstanz) |
| Rest | Wasser |

Als aw-Wert ergibt sich 0,920. Die Viskosität bei 20 °C beträgt ca. 68.800 cP.

Obwohl die in diesem Beispiel beschriebene erfindungsgemäße Zusammensetzung eine erheblich höhere Viskosität aufweist als die vorstehend als bevorzugt empfohlene, sind solche Zusammensetzungen fließfähig und mit üblichen lebensmitteltechnologischen Geräten pump- und dosierbar.

## Patentansprüche

1. Fliessfähige Gelatinezusammensetzung, umfassend eine wässrige Flüssigkeit, darin dispergierte Gelatinegelpartikel und/oder gelöstes Gelatinehydrolysat und einen oder mehrere Zuckerbestandteile, wobei die Summe der Gehalte an Gelatine, Gelatinehydrolysat und Zuckerbestandteil(e) so gewählt ist, dass die Wasseraktivität (aw-Wert) der Zusammensetzung kleiner oder gleich 0,97 ist.

2. Zusammensetzung nach Anspruch 1, **dadurch gekennzeichnet, dass** die Wasseraktivität (aw-Wert) 0,93 oder weniger beträgt.

3. Zusammensetzung nach Anspruch 1 oder 2, **dadurch gekennzeichnet, dass** der Gehalt an Gelatinegelpartikeln (Trockenmasse) 20 bis 40 Gew.% beträgt.

4. Zusammensetzung nach einem der Ansprüche 1 bis 3, **dadurch gekennzeichnet, dass** als Zuckerbestandteil ein oder mehrere Saccharide enthalten sind, wobei der Saccharidgehalt 30 Gew.% oder mehr beträgt.

5. Zusammensetzung nach einem der Ansprüche 1 bis 4, **dadurch gekennzeichnet, dass** die Gelatinegelpartikel im gequollenen Zustand eine mittlere Partikelgröße von ca. 0,01 bis ca. 3 mm, insbesondere 0,1 bis 1 mm aufweisen.

6. Zusammensetzung nach einem der Ansprüche 1 bis 5, **dadurch gekennzeichnet, dass** der Gehalt an Gelatinehydrolysat 20 bis 60 Gew.% beträgt.

7. Zusammensetzung nach Anspruch 6, **dadurch gekennzeichnet, dass** das mittlere Molekulargewicht (MW) des Gelatinehydrolysats ca. 1.000 bis ca. 20.000 Da beträgt.

8. Zusammensetzung nach Anspruch 6 oder 7, **dadurch gekennzeichnet, dass** als Zuckerbestandteil ein oder mehrere Saccharide enthalten sind, wobei der Saccharidgehalt 10 Gew.% oder mehr beträgt.

9. Zusammensetzung nach einem der Ansprüche 1 bis 8, **dadurch gekennzeichnet, dass** das Saccharid ausgewählt ist aus Mono-, Di- und/oder Oligosacchariden.

10. Zusammensetzung nach einem der Ansprüche 1 bis 9, **dadurch gekennzeichnet, dass** als Zuckerbestandteil ein oder mehrere Alditole enthalten sind.

11. Zusammensetzung nach einem der Ansprüche 1 bis 10, **dadurch gekennzeichnet, dass** die Zusammensetzung einen Anteil an einer oder mehreren Genußsäuren umfaßt.

12. Zusammensetzung nach Anspruch 11, **dadurch gekennzeichnet, dass** der pH-Wert der Zusammensetzung kleiner 5 beträgt.

13. Zusammensetzung nach Anspruch 12, **dadurch gekennzeichnet, dass** der pH-Wert der Zusammensetzung ca. 3 bis ca. 4,5 beträgt.

14. Zusammensetzung nach einem der Ansprüche 1 bis 13, **dadurch gekennzeichnet, dass** die Zusammensetzung eine Viskosität von höchstens 30.000 cP aufweist.

15. Verwendung einer Zusammensetzung nach einem der Ansprüche 1 bis 14 als Lebensmittelvorprodukt.

16. Verwendung einer Zusammensetzung nach einem der Ansprüche 1 bis 14 bei der Herstellung von Kapseln für die pharmazeutische Industrie.

## Claims

1. Free-flowing gelatin composition, comprising an aqueous liquid, gelatin gel particles dispersed therein and/or gelatin hydrolysate dissolved therein and one or more sugar components, wherein the sum of the contents of gelatin, gelatin hydrolysate and sugar component(s) is selected such that the water activity (aw value) of the composition is less than or equal to 0.97.

2. Composition according to claim 1, **characterized in that** the water activity (aw value) is 0.93 or less.

3. Composition according to claim 1 or 2, **characterized in that** the gelatin gel particle (dry mass) content is 20 to 40 wt.%.

4. Composition according to one of claims 1 to 3, **characterized in that** as a sugar component one or more saccharides are contained, wherein the saccharide content is 30 wt.% or more.

5. Composition according to one of claims 1 to 4, **characterized in that** the gelatin gel particles in the swollen state have a mean particle size of ca. 0.01 to ca. 3 mm, in particular 0.1 to 1 mm.

6. Composition according to one of claims 1 to 5, **characterized in that** the gelatin hydrolysate content is 20 to 60 wt.%.

7. Composition according to claim 6, **characterized in that** the mean molecular weight (MW) of the gelatin hydrolysate is ca. 1,000 to ca. 20,000 Da.

8. Composition according to claim 6 or 7, **characterized in that** as a sugar component one or more saccharides are contained, wherein the saccharide content is 10 wt.% or more.

9. Composition according to one of claims 1 to 8, **characterized in that** the saccharide is selected from mono-, di- and/or oligosaccharides.

10. Composition according to one of claims 1 to 9, **characterized in that** as a sugar component one or more alditols are contained.

11. Composition according to one of claims 1 to 10, **characterized in that** the composition comprises a fraction of one or more edible acids.

12. Composition according to claim 11, **characterized in that** the pH value of the composition is lower than 5.

13. Composition according to claim 12, **characterized in that** the pH value of the composition is ca. 3 to ca. 4.5.

14. Composition according to one of claims 1 to 13, **characterized in that** the composition has a viscosity of at most 30,000 cP.

15. Use of a composition according to one of claims 1 to 14 as a food precursor.

16. Use of a composition according to one of claims 1 to 14 in the manufacture of capsules for the pharmaceutical industry.

## Revendications

1. Composition de gélatine fluide, comportant un liquide aqueux, des particules de gel de gélatine dispersées et/ou un hydrolysat de gélatine dissous dans celui-ci et un ou plusieurs composants sucrés, la somme des teneurs en gélatine, hydrolysat de gélatine et composant(s) sucré(s) étant choisie de telle sorte que l'activité de l'eau (valeur aw) dans la composition est inférieure ou égale à 0,97.

2. Composition selon la revendication 1, **caractérisée en ce que** l'activité de l'eau (valeur aw) est de 0,93 ou moins.

3. Composition selon la revendication 1 ou 2, **caractérisée en ce que** la teneur en particules de gel de gélatine (masse sèche) est de 20 à 40 % en poids.

4. Composition selon l'une des revendications 1 à 3, **caractérisée en ce qu'**un ou plusieurs saccharides sont présents en tant que composant sucré, la teneur en saccharides étant de 30 % en poids ou plus.

5. Composition selon l'une des revendications 1 à 4, **caractérisée en ce que** les particules de gel de gélatine à l'état gonflé ont une dimension moyenne de particule d'environ 0,01 à environ 3 mm, notamment de 0,1 à 1 mm.

6. Composition selon l'une des revendications 1 à 5, **caractérisée en ce que** la teneur en hydrolysat de gélatine est de 20 à 60 % en poids.

7. Composition selon la revendication 6, **caractérisée en ce que** la masse moléculaire moyenne (MW) de l'hydrolysat de gélatine est d'environ 1 000 à environ 20 000 Da.

8. Composition selon la revendication 6 ou 7, **caractérisée en ce qu'**un ou plusieurs saccharides sont présents en tant que composant sucré, la teneur en saccharides étant de 10 % en poids ou plus.

9. Composition selon l'une des revendications 1 à 8, **caractérisée en ce que** le saccharide est choisi parmi les monosaccharides, les disaccharides et/ou les oligosaccharides.

10. Composition selon l'une des revendications 1 à 9, **caractérisée en ce qu'**un ou plusieurs alditols sont présents en tant que composant sucré.

11. Composition selon l'une des revendications 1 à 10, **caractérisée en ce que** la composition comprend une proportion d'un ou plusieurs acides alimentaires.

12. Composition selon la revendication 11, **caractérisée en ce que** le pH de la composition est inférieur à 5.

13. Composition selon la revendication 12, **caractérisée en ce que** le pH de la composition est d'environ 3 à environ 4,5.

14. Composition selon l'une des revendications 1 à 13, **caractérisée en ce que** la composition présente une viscosité maximale de 30 000 cP.

15. Utilisation d'une composition selon l'une des revendications 1 à 14 comme semi-produit alimentaire.

16. Utilisation d'une composition selon l'une des revendications 1 à 14 dans la préparation de capsules destinées à l'industrie pharmaceutique.
